# EUROPÄISCHE PATENTSCHRIFT

(11) **EP 1 620 044 B1**
(45) Veröffentlichungstag und Bekanntmachung des Hinweises auf die Patenterteilung: **19.03.2008**
(21) Anmeldenummer: 03714601.6
(22) Anmeldetag: 28.04.2003
(51) Int. Cl.: A61F 2/44, A61F 2/30

(54) **ZWISCHENWIRBELIMPLANTAT**
INTERVERTEBRAL IMPLANT
IMPLANT INTERVERTEBRAL

(43) Veröffentlichungstag der Anmeldung: 01.02.2006
(73) Patentinhaber: Synthes GmbH, 4436 Oberdorf (CH)
(72) Erfinder: GÜTLIN, Michael, CH-4133 Pratteln (CH); SCHÄR, Manuel, CH-4132 Muttenz (CH); LECHMANN, Beat, CH-2544 Bettlach (CH)
(74) Vertreter: Lusuardi, Werther
(86) Internationale Anmeldenummer: PCT/CH2003/000273
(87) Internationale Veröffentlichungsnummer: WO 2004/096103

(56) Entgegenhaltungen:
- EP-A- 1 080 703
- EP-A- 1 219 266
- DE-A- 19 509 317
- DE-A- 19 622 827
- US-A1- 2002 161 441

## Beschreibung

Die Erfindung bezieht sich auf ein Zwischenwirbelimplantat gemäss dem Oberbegriff des Patentanspruchs 1.

Aus der DE-A 196 22 827 ist ein gattungsgemässes Zwischenwirbelimplantat bekannt. Dabei ist der mittlere, als Ring mit Innengewinde ausgebildete Implantatteil lediglich mit dem oberen, endständigen Implantatteil gewindemässig verbunden. Gegenüber dem unteren, endständigen Implantatteil ist der Ring sowohl axial als auch rotativ frei beweglich. Der untere, endständige Implantatteil dient dem Ring nur als axialer Anschlag, d.h. als Abstützfläche gegen welche der Ring verdreht werden kann, so dass das obere, endständige Implantatteil axial bewegt werden kann (Mutter/Spindel-Antrieb). Die beiden endständigen Implantatteil sind somit lose gegeneinander montiert, was sich auch darin zeigt, dass zu ihrer axialen Sicherung eine radiale Gewindebohrung in einem der beiden Implantatteile vorgesehen ist, durch welche eine verspannbare Klemmschraube geführt ist. Somit hat dieses bekannte Zwischenwirbelimplantat den Nachteil, dass es prä- und intraoperativ ungesichert ist und auseinanderfallen kann. Ein Herunterfallen eines Implantatteils im Operationssaal würde aus Sterilitätsgründen zu einer erheblichen Zeitverzögerung führen.

Aus der EP-A 1 219 266 ULRICH eine teleskopierbare Zwischenwirbelprothese mit einem Gewindering bekannt, der eine Kegelradverzahnung aufweist, welche von radial her über ein Kegelrad-Ritzel verdreht werden kann. Nachteilig bei dieser bekannten Vorrichtung ist der Umstand, dass der Gewindering nicht mit einem der Hülsenteile axial fest verbunden ist. Dadurch ist das Implantat weniger stabil konstruiert und zudem besteht die Notwendigkeit eines speziellen Instrumentes um die Distraktion des Implantates herbeizuführen. Ein weiterer Nachteil dieses bekannten Implantates besteht in der verwendeten Kegelradverzahnung, welche wenig "verzeihend" und somit nachteilig ist. Im Fall der Kegelradverzahnung kann das Antriebsinstrument bei einem geringen axialen Verschieben bereits nicht mehr seine Funktion wahrnehmen und der Chirurg muss in-situ das Instrument neu plazieren.

Hier will die Erfindung Abhilfe schaffen. Der Erfindung liegt die Aufgabe zugrunde, ein Zwischenwirbelimplantat zu schaffen, welches unter Beibehaltung einer geringstmöglichen Bauhöhe ein kompaktes gegen Auseinanderfallen der einzelnen Teile gesichertes Ganzes bildet und als vormontiertes Set dem Chirurgen zur Verfügung gestellt werden kann.

Die Erfindung löst die gestellte Aufgabe mit einem Zwischenwirbelimplantat, welches die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank des erfindungsgemässen Zwischenwirbelimplantates ein vereinfachtes Handling möglich ist, welches einerseits die Sicherheit erhöht und anderseits den Zeitaufwand für die Implantation reduziert.

Bei einer speziellen Ausführungsform ist der Ring des Zwischenwirbelimplantates mittels einer Klippverbindung am unteren Implantatteil axial fest aber rotativ beweglich befestigt. So bleibt der Ring für den Antrieb sicher in Position, d.h. der Ring kann axial nicht weichen.
Vorzugsweise wird die Klippverbindung aus einem torusförmigen Hinterschnitt im Ring und einem damit korrespondierenden ringförmigen Wulst am freien Ende des Mantels des unteren Implantatteils gebildet. Dadurch wird die Rotation des Ringes solchermassen gesichert, dass die Rotation um das gleiche Zentrum erfolgt wie die beiden hülsenförmigen Implantatteile.

Erfindungsgemäß weist der Ring eine untere, dem unteren Implantatteil zugewandte Kreisringfläche und eine obere, dem oberen Implantatteil zugewandte Kreisringfläche auf, wobei die untere Kreisringfläche einen Zahnkranz aufweist, der geeignet ist einen damit korrespondierenden Ritzel aufzunehmen. Diese Ausführung gestattet durch radiale Rotation des den Ritzel tragenden Instrumentes eine axiale Rotation des Ringes. Gegenüber den bekannten Implantaten, bei denen der Ring tangential bewegt werden muss, ergibt sich der Vorteil des geringeren Platzbedarfs für das Verdrehinstrument sowie der höheren Geschwindigkeit, weil stufenlos verdreht werden kann ohne die Notwendigkeit das Verdrehinstrument immer wieder in neue Bohrungen des Ringes einführen zu müssen.

Bei einer weiteren Ausführungsform weist der Mantel des unteren Implantatteils eine radiale, vorzugsweise durchgehende Vertiefung auf, welche geeignet ist einen Ritzel gegenüber dem Zahnkranz zu positionieren und abzustützen. Dadurch ergibt sich die vorteilhafte Möglichkeit, nach erfolgter Distraktion der Wirbel, durch die durchgehende Vertiefung Knochenspäne in die Kavität des unteren Implantatteils einführen zu können.

Bei einer weiteren Ausführungsform weist der kreiszylindrische Schaft (6) des oberen Implantatteils - zur Sicherung gegen Rotation des oberen gegenüber dem unteren Implantatteil - einen parallel zur Rotationsachse verlaufenden Führungsschlitz und der Mantel des unteren Implantatteils an seiner Innenseite ein nach Innen vorragendes, in den Führungsschlitz eingreifendes Führungselement (17) auf, welches vorzugsweise durch einen radialen Durchbruch im Mantel einbringbar ist. Dadurch wird das gegenseitige Rotieren der beiden Implantatteile vermieden. Ein gegenseitiges Rotieren würde den Kontakt des Implantates zu den Endplatten und die lordosierenden Winkel beeinträchtigen.

Bei einer weiteren Ausführungsform weist der kreiszylindrische Schaft (6) des oberen Implantatteils (5) einen geringeren Umfang auf als die hohlzylindrische Kavität (3) des unteren Implantatteils (2), so dass sich die beiden Bauteile nicht berühren.

Erfindungsgemäß ist das Aussengewinde des kreiszylindrischen Schaftes und das Innengewinde des Ringes selbsthemmend ausgebildet. Durch die Selbsthemmung des Gewindes kann das Implantat auf eine beliebige Höhe distrahiert werden, ohne dass dazu eine zusätzliche Arretierung notwendig wäre.
Bei einer weiteren Ausführungsform liegt die Steigung des Aussengewindes und des Innengewindes im Bereich von 0,5 bis 1,0 mm, vorzugsweise von 0,6 - 0,8 mm. Grössere Steigungen, z.B. im Bereich von 2 mm, zeigten intraoperative Nachteile, da die Distraktion sich als zu grob herausstellte und kleinere Steigungen hatten ein zeitlich längeres Drehen zur Folge.

Bei einer weiteren Ausführungsform sind sowohl das Aussengewinde als auch das Innengewinde als Rechtsgewinde ausgebildet. Es ergibt sich daraus der Vorteil, dass das Instrument, das den Ritzel trägt, in üblicher Weise, wie ein normaler Schraubenzieher rechtshändig gedreht werde kann, um das Implantat zu distrahieren.

Bei einer weiteren Ausführungsform weist ist sowohl das Aussengewinde als auch das Innengewinde als mehrgängiges, vorzugsweise als zweigängiges Gewinde ausgebildet. Bei einem zweigängigen Gewinde wird pro Umdrehung des Ringes ein doppelter Hub erzielt. Dadurch ergibt sich eine schnellere und einfachere Handhabung des Implantates, was die Operationszeit verkürzt und den Patienten schont.

Bei einer weiteren Ausführungsform sind die beiden Appositionsteile als quer zur Rotationsachse stehende plattenförmige Elemente mit einer zur Anlage an die Deckfläche des angrenzenden Wirbels bestimmten Appositionsfläche ausgebildet.

Bei einer weiteren Ausführungsform sind die Appositionsflächen nicht orthogonal zur Rotationsachse angeordnet sondern bilden vorzugsweise einen Winkel 83° bis 85° mit der Rotationsachse. Dadurch ergibt sich eine bessere Anpassung an die anatomischen Verhältnisse. Vorzugsweise schliessen die beiden Appositionsflächen einen Winkel von 10° bis 14° untereinander ein. Dadurch gelingt es den anatomischen Lordosewinkel sicherzustellen.
Bei einer weiteren Ausführungsform ist mindestens eine der beiden Appositionsflächen, vorzugsweise diejenige des oberen Implantatteils nach aussen gewölbt. Dadurch ergibt sich eine optimale Anpassung an den Wirbelkörper. Die Appositionsfläche des unteren Implantatteils ist vorzugsweise im wesentlichen eben ausgebildet.

Bei einer weiteren Ausführungsform ist der der Zahnkranz geradeverzahnt. Gegenüber einem kegeligen/schrägverzahnten Zahnkranz ist die Geradverzahnung "verzeihender".

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht des Zwischenwirbelimplantates im zusammengesetzten Zustand;
Fig. 2 eine perspektivische Ansicht des Zwischenwirbelimplantates im demontierten Zustand;
Fig. 3 einen Längsschnitt durch die Rotationsachse des Zwischenwirbelimplantates nach Fig. 1;
Fig. 4 eine Vorderansicht des Zwischenwirbelimplantates nach Fig. 1;
Fig. 5 einen Längsschnitt durch die Rotationsachse des Zwischenwirbelimplantates nach Fig. 1 im demontierten Zustand; und
Fig. 6 eine Vorderansicht des Zwischenwirbelimplantates nach Fig. 1 im demontierten Zustand.

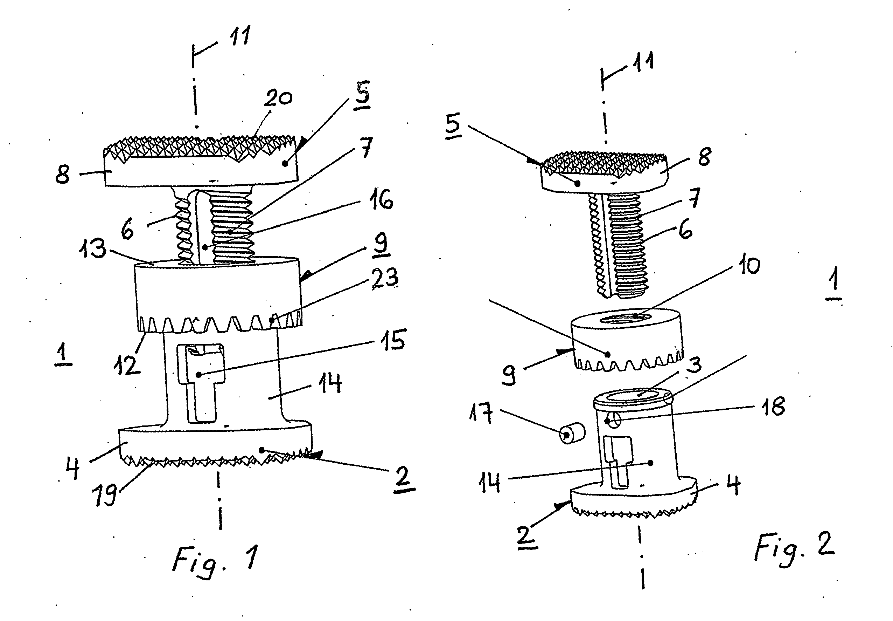

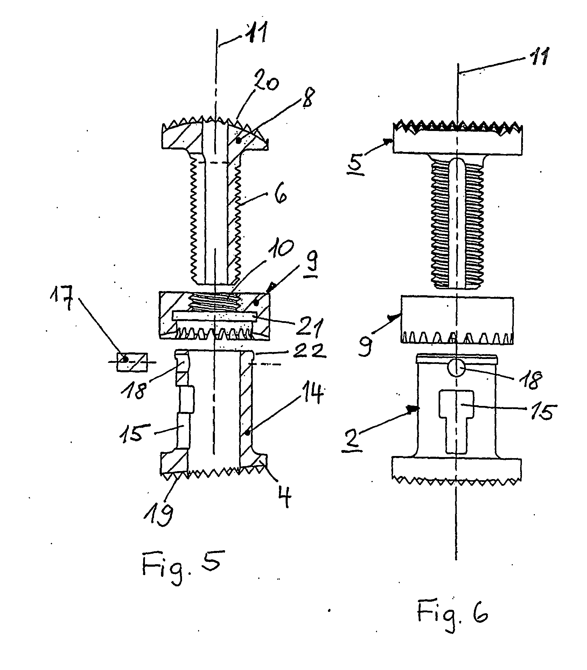

Das in Fig. 1 - 6 dargestellte Zwischenwirbelimplantat 1 besteht im wesentlichen aus einem unteren Implantatteil 2, einem oberen Implantatteil 5 und einem zwischen den beiden Implantatteilen 2,5 angeordneten Ring 9. Der untere Implantatteil 2, besitzt eine hohlzylindrische Kavität 3 mit einem Mantel 14 und einer Rotationsachse 11 und einem zur Anlage an die Deckfläche des angrenzenden unteren Wirbels bestimmten Appositionsteil 4. Der obere Implantatteil 5 besitzt einen im wesentlichen kreiszylindrischen Schaft 6 mit Aussengewinde 7 und Rotationsachse 11, der sich in die Kavität 3 des unteren Implantatteils 2 erstreckt und einem zur Anlage an die Deckfläche des angrenzenden oberen Wirbels bestimmten Appositionsteil 8. Die beiden Implantatteile sind gegen Rotation um ihre Rotationsachse 11 gesichert.

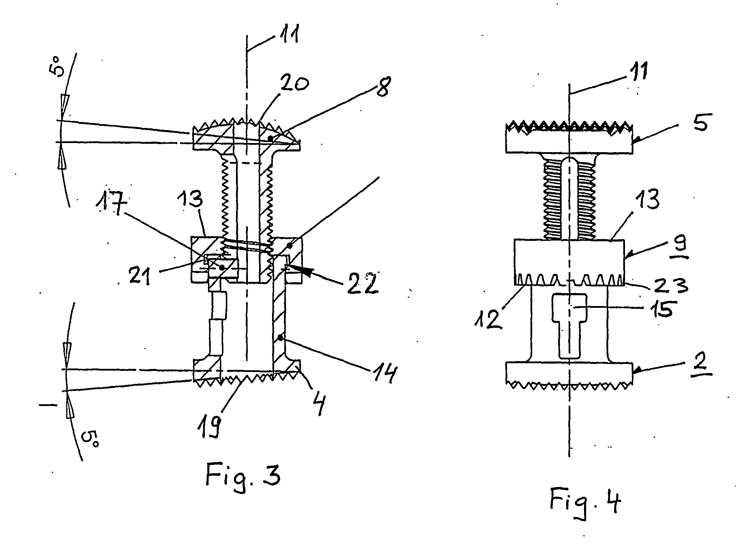

Der Ring 9 besitzt ein Innengewinde 10, welches mit dem Aussengewinde 7 des oberen Implantatteils 5 zusammenwirkt, wobei die beiden Implantatteile 2,5 und der Ring 9 koaxial längs ihrer gemeinsamen Rotationsachse 11 angeordnet sind, so dass durch Verdrehen des Ringes 9 auf dem Aussengewinde 7 des Schaftes 6 der Abstand zwischen den beiden Appositionsteilen 4,8 stufenlos veränderbar ist Der Ring 9 ist mittels einer Klippverbindung axial fest aber rotativ beweglich mit dem unteren Implantatteil 2 verbunden.
Wie in Fig. 3 dargestellt besteht die Klippverbindung aus einem torusförmigen Hinterschnitt 21 im Ring 9 und einem damit korrespondierenden ringförmigen Wulst 22 am freien Ende des Mantels 14 des unteren Implantatteils 2.

Der Ring 9 besitzt eine untere, dem unteren Implantatteil 2 zugewandte Kreisringfläche 12 und eine obere, dem oberen Implantatteil zugewandte Kreisringfläche 13, wobei die untere Kreisringfläche 12 einen geradverzahnten Zahnkranz 23 aufweist, der geeignet ist einen damit korrespondierenden Ritzel aufzunehmen.

Der Mantel 14 des unteren Implantatteils 2 besitzt eine radiale, durchgehende Vertiefung 15, welche geeignet ist einen (zeichnerisch nicht dargestellten) Ritzel gegenüber dem Zahnkranz 23 zu positionieren und abzustützen.

Zur Sicherung gegen Rotation des oberen Implantatteils 5 gegenüber dem unteren Implantatteil 2, weist der kreiszylindrische Schaft 6 des oberen Implantatteils 5 einen parallel zur Rotationsachse 11 verlaufenden Führungsschlitz 16 und der Mantel 14 des unteren Implantteils 2 an seiner Innenseite ein nach Innen vorragendes, in den Führungsschlitz 16 eingreifendes Führungselement 17 (Fig. 2) auf, welches durch einen radialen Durchbruch 18 im Mantel 14 einbringbar ist.
Das Aussengewinde 7 des kreiszylindrischen Schaftes 6 und das Innengewinde 10 des Ringes 9 sind selbsthemmend ausgebildet. Die Steigung des Aussengewindes 7 und des Innengewindes 10 beträgt 0,7 mm. Sowohl das Aussengewinde 7 als auch das Innengewinde 10 sind als zweigängiges Rechtsgewinde ausgebildet.

Wie in Fig. 3 dargestellt sind die beiden Appositionsteile 4,8 als quer zur Rotationsachse 11 stehende plattenförmige Elemente mit einer zur Anlage an die Deckfläche des angrenzenden Wirbels bestimmten Appositionsfläche 19,20 ausgebildet, wobei die Appositionsflächen 19,20 nicht orthogonal zur Rotationsachse 11 angeordnet sind sondern einen Winkel von 85 ° mit der Rotationsachse 11 einschliessen. Die beiden Appositionsflächen 19,20 schliessen somit einen Winkel von 10° untereinander ein. Die obere Appositionsfläche 20 ist - wie in Fig. 3 dargestellt - nach aussen gewölbt, währenddem die Appositionsfläche 19 des unteren Implantatteils im wesentlichen eben ausgebildet ist.

Das Zwischenwirbelimplantat kann aus den üblichen Implantatmaterialien hergestellt werden. Vorzugsweise wird röntgentrahlendurchlässiges PEEK verwendet, um eine Beurteilung der Fusion mittels Röntgenstrahlen zu gestatten.
Das Zwischenwirbelimplantat eignet sich insbesondere als Wirbelkörperersatz im cervikalen und höheren thorakalen Bereich der Wirbelsäule.

## Patentansprüche

1. Zwischenwirbelimplantat (1) mit
A) einem unteren Implantatteil (2), welches eine hohlzylindrische Kavität (3) mit einem Mantel (14) und einer Rotationsachse (11) und einem zur Anlage an die Deckfläche des angrenzenden unteren Wirbels bestimmten Appositionsteil (4);
B) einem oberen Implantatteil (5) mit einem im wesentlichen kreiszylindrischen Schaft (6) mit Aussengewinde (7) und Rotationsachse (11), der sich in die Kavität (3) des unteren Implantatteils (2) erstreckt und einem zur Anlage an die Deckfläche des angrenzenden oberen Wirbels bestimmten Appositionsteil (8); wobei
C) die beiden Implantatteile (2,5) gegen Rotation um ihre Rotationsachse (11) gesichert sind; sowie
D) einem zwischen den beiden Implantatteilen (2,5) angeordneten Ring (9) mit Innengewinde (10), welches mit dem Aussengewinde (7) oberen Implantatteils (5) zusammenwirkt; wobei
E) die beiden Implantatteile (2,5) und der Ring (9) koaxial längs ihrer gemeinsamen Rotationsachse (11) angeordnet sind, so dass
F) durch Verdrehen des Ringes (9) auf dem Aussengewinde (7) des Schaftes (6) der Abstand zwischen den beiden Appositionsteilen (4,8) stufenlos veränderbar ist;
**dadurch gekennzeichnet, dass**
G) der Ring (9) axial fest aber rotativ beweglich mit dem unteren Implantatteil (2) verbunden ist; und
H) der Ring (9) eine untere, dem unteren Implantatteil (2) zugewandte Kreisringfläche (12) und eine obere, dem oberen Implantatteil zugewandte Kreisringfläche (13) aufweist, wobei
I) die untere Kreisringfläche (12) einen Zahnkranz (23) aufweist, der geeignet ist einen damit korrespondierenden Ritzel aufzunehmen; und
J) das Aussengewinde (7) des kreiszylindrischen Schaftes (6) und das Innengewinde (10) des Ringes (9) selbsthemmend ausgebildet sind.

2. Zwischenwirbelimplantat (1) nach Anspruch 1, **dadurch gekennzeichnet, dass** der Ring (9) mittels einer Klippverbindung am unteren Implantatteil (2) axial fest aber rotativ beweglich befestigt ist.

3. Zwischenwirbelimplantat (1) nach Anspruch 2, **dadurch gekennzeichnet, dass** die Klippverbindung aus einem torusförmigen Hinterschnitt (21) im Ring (9) und einem damit korrespondierenden ringförmigen Wulst (22) am freien Ende des Mantels (14) des unteren Implantatteils (2) gebildet wird.

4. Zwischenwirbelimplantat (1) nach einem der Ansprüche 1 bis 3, **dadurch gekennzeichnet, dass** der Mantel (14) des unteren Implantatteils (2) eine radiale Vertiefung (15) aufweist, welche geeignet ist einen Ritzel gegenüber dem Zahnkranz (23) zu positionieren und abzustützen.

5. Zwischenwirbelimplantat (1) nach Anspruch 4, **dadurch gekennzeichnet, dass** die Vertiefung (15) durchgehend ist.

6. Zwischenwirbelimplantat (1) nach einem der Ansprüche 1 bis 5, **dadurch gekennzeichnet, dass** zur Sicherung gegen Rotation (6) des oberen (5) gegenüber dem unteren Implantatteil (2), der kreiszylindrische Schaft (6) des oberen Implantatteils (5) einen parallel zur Rotationsachse (11) verlaufenden Führungsschlitz (16) aufweist und der Mantel (14) des unteren Implantatteils (2) an seiner Innenseite ein nach Innen vorragendes, in den Führungsschlitz (16) eingreifendes Führungselement (17) aufweist, welches vorzugsweise durch einen radialen Durchbruch (18) im Mantel (14) einbringbar ist.

7. Zwischenwirbelimplantat (1) nach einem der Ansprüche 1 bis 6, **dadurch gekennzeichnet, dass** der kreiszylindrische Schaft (6) des oberen Implantatteils (5) einen geringeren Umfang aufweist als die hohlzylindrische Kavität (3) des unteren Implantatteils (2), so dass sich die beiden Bauteile nicht berühren.

8. Zwischenwirbelimplantat (1) nach einem der Ansprüche 1 bis 7, **dadurch gekennzeichnet, dass** die Steigung des Aussengewindes (7) und des Innengewindes (10) im Bereich von 0,5 bis 1,0 mm, vorzugsweise von 0,6 - 0,8 mm liegt.

9. Zwischenwirbelimplantat (1) nach einem der Ansprüche 1 bis 8, **dadurch gekennzeichnet, dass** sowohl das Aussengewinde (7) als auch das Innengewinde (10) als Rechtsgewinde ausgebildet sind.

10. Zwischenwirbelimplantat (1) nach einem der Ansprüche 1 bis 9, **dadurch gekennzeichnet, dass** sowohl das Aussengewinde (7) als auch das Innengewinde (10) als mehrgängiges, vorzugsweise als zweigängiges Gewinde ausgebildet sind.

11. Zwischenwirbelimplantat (1) nach einem der Ansprüche 1 bis 10, **dadurch gekennzeichnet, dass** die beiden Appositionsteile (4,8) als quer zur Rotationsachse (11) stehende plattenförmige Elemente mit einer zur Anlage an die Deckfläche des angrenzenden Wirbels bestimmten Appositionsfläche (19,20) ausgebildet sind.

12. Zwischenwirbelimplantat (1) nach Anspruch 11, **dadurch gekennzeichnet, dass** die Appositionsflächen (19,20) nicht orthogonal zur Rotationsachse (11) angeordnet sind und vorzugsweise einen Winkel 83° bis 85 ° mit der Rotationsachse (11) einschliessen.

13. Zwischenwirbelimplantat (1) nach Anspruch 12, **dadurch gekennzeichnet, dass** die beiden Appositionsflächen (19,20) einen Winkel von 10° bis 14° untereinander einschliessen.

14. Zwischenwirbelimplantat (1) nach einem der Ansprüche 11 bis 13, **dadurch gekennzeichnet, dass** mindestens eine der beiden Appositionsflächen (19,20), vorzugsweise diejenige des oberen Implantatteils (5) nach aussen gewölbt ist.

15. Zwischenwirbelimplantat (1) nach einem der Ansprüche 11 bis 13, **dadurch gekennzeichnet, dass** die Appositionsfläche (19) des unteren Implantatteils, im wesentlichen eben ausgebildet ist.

16. Zwischenwirbelimplantat (1) nach einem der Ansprüche 4 bis 15, **dadurch gekennzeichnet, dass** der Zahnkranz (23) geradeverzahnt ist.

## Claims

1. Intervertebral implant (1) with
A) a lower implant section (2), that has a hollow cylindrical cavity (3) with a jacket (14) and a rotation axis (11) and an apposition section (4) intended for placement on the top surface of the neighbouring lower vertebra;
B) an upper implant section (5) with a shaft (6) of generally circular-cylindrical design with external thread (7) and rotation axis (11), reaching into the cavity (3) of the lower implant section (2) and an apposition section (8) intended for placement on the top surface of the neighbouring upper vertebra; wherein
C) the two implant sections (2,5) are secured against rotation around their rotation axis (11); and
D) a ring (9) with internal thread (10) positioned between the two implant sections (2,5) which interacts with the external thread (7) of the upper implant section (5); wherein
E) the two implant sections (2,5) and the ring (9) are arranged coaxial along their common rotation axis (11), so that
F) by twisting of the ring (9) on the external thread (7) of the shaft (6) the distance between the two apposition sections (4,8) can be adjusted continuously;
**characterised in that**
G) the ring (9) is connected to the lower implant section (2) fixed in an axial but moveable in a rotation direction; and
H) the ring (9) is provided with a lower circular ring surface (12) facing the lower implant section (2) and an upper circular ring surface (13) facing the upper implant section, wherein
I) the lower circular ring surface (12) is provided with a crown gear (23) which is suitable for receiving a corresponding pinion; and
J) the external thread (7) of the circular-cylindrical shaft (6) and the internal thread (10) of the ring (9) have a self-locking feature.

2. Intervertebral implant (1) according to Claim 1, **characterised in that** the ring (9) is attached by means of a clip connection to the lower implant section (2) fixed in an axial but moveable in a rotation direction.

3. Intervertebral implant (1) according to Claim 2, **characterised in that** the clip connection is formed from a toroid undercut (21) in the ring (9) and a corresponding ring-shaped bead (22) at the free end of the jacket (14) of the lower implant section (2).

4. Intervertebral implant (1) according to one of the claims 1 to 3, **characterised in that** the jacket (14) of the lower implant section (2) is provided with a radial depression (15), which is suitable for positioning and supporting a pinion in relation to the crown gear (23).

5. Intervertebral implant (1) according to claim 4, **characterised in that** the depression (15) is continuous.

6. Intervertebral implant (1) according to one of the claims 1 to 5, **characterised in that**, to secure against rotation (6) of the upper (5) in relation to the lower implant section (2), the circular-cylindrical shaft (6) of the upper implant section (5) is provided with a guide aperture (16) running parallel to the rotation axis (11), and the jacket (14) of the lower implant section (2) is provided at its inner side with a guiding element (17) protruding inwards and engaging in the guide aperture (16), which can preferably be inserted by a radial breach (18) in the jacket (14).

7. Intervertebral implant (1) according to one of the claims 1 to 6, **characterised in that** the circular-cylindrical shaft (6) of the upper implant section (5) has a circumference less than the hollow cylindrical cavity (3) of the lower implant section (2), so that the two sections do not come in contact.

8. Intervertebral implant (1) according to one of the claims 1 to 7, **characterised in that** the gradient of the external thread (7) and of the internal thread (10) is within the range 0.5 to 1.0 mm, and preferably 0.6 - 0.8 mm.

9. Intervertebral implant (1) according to one of the claims 1 to 8, **characterised in that** both the external thread (7) and the internal thread (10) are designed as right-hand thread.

10. Intervertebral implant (1) according to one of the claims 1 to 9 **characterised in that** both the external thread (7) and the internal thread (10) are formed as multi-flighted, preferably as 2-flighted thread.

11. Intervertebral implant (1) according to one of the claims 1 to 10, **characterised in that** the two apposition sections (4,8) are designed as plate-shaped elements standing perpendicular to the rotation axis (11) with an apposition surface (19,20) intended for placement on the top surface of the neighbouring vertebra.

12. Intervertebral implant (1) according to Claim 11, **characterised in that** the apposition surfaces (19,20) are not arranged orthogonal to the rotation axis (11) and preferably form an angle 83° to 85 ° to the rotation axis (11).

13. Intervertebral implant (1) according to Claim 12, **characterised in that** the two apposition surfaces (19,20) form an angle of 10° to 14° to each other.

14. Intervertebral implant (1) according to one of the claims 11 to 13, **characterised in that** at least one of the two apposition surfaces (19,20), preferably that of the upper implant section (5) is arched on the outside.

15. Intervertebral implant (1) according to one of the claims 11 to 13, **characterised in that** the apposition surface (19) of the lower implant section is generally designed as a flat surface.

16. Intervertebral implant (1) according to one of the claims 4 to 15, **characterised in that** the crown gear (23) is straight toothed.

## Revendications

1. Implant intervertébral (1) comprenant
A) une partie inférieure de l'implant (2), qui présente une cavité en forme de cylindre creux (3) avec une enveloppe (14) et un axe de rotation (11) et une partie d'apposition (4) servant à l'appui sur la surface de recouvrement de la vertèbre inférieure adjacente ;
B) une partie supérieure de l'implant (5) présentant une tige essentiellement en forme de cylindre circulaire (6), pourvue d'un filetage (7) et d'un axe de rotation (11), qui s'étend dans la cavité (3) de la partie inférieure de l'implant (2), et une partie d'apposition (8) servant à l'appui sur la surface de recouvrement de la vertèbre supérieure adjacente ; dans lequel
C) les deux parties d'implant (2, 5) sont bloquées en rotation autour de leur axe de rotation (11) ; et
D) une bague (9) disposée entre les deux parties d'implant (2, 5), pourvue d'un taraudage (10) qui agit conjointement avec le filetage (7) de la partie supérieure de l'implant (5) ; dans lequel
E) les deux parties d'implant (2, 5) et la bague (9) sont disposées coaxialement le long de leur axe de rotation commun (11) de sorte que
F) lors d'une rotation de la bague (9) sur le filetage (7) de la tige (6), la distance entre les deux parties d'apposition (4, 8) est modifiable de manière progressive ;
**caractérisé en ce que**
G) la bague (9) est reliée à la partie inférieure de l'implant (2) de manière fixe sur le plan axial mais mobile sur le plan rotatif ; et
H) la bague (9) présente une surface annulaire inférieure (12) tournée vers la partie inférieure de l'implant (2) et une surface annulaire supérieure (13) tournée vers la partie supérieure de l'implant, dans lequel
I) la surface annulaire inférieure (12) présente une couronne dentée (23) qui est appropriée pour recevoir un pignon correspondant à celle-ci ; et
J) le filetage (7) de la tige en forme de cylindre circulaire (6) et le taraudage (10) de la bague (9) sont conçus sous une forme autobloquante.

2. Implant intervertébral (1) selon la revendication 1, **caractérisé en ce que** la bague (9) est fixée à la partie inférieure de l'implant (2) de manière fixe sur le plan axial mais mobile sur le plan rotatif au moyen d'un assemblage par encliquetage.

3. Implant intervertébral (1) selon la revendication 2, **caractérisé en ce que** l'assemblage par encliquetage est formé par une contre-dépouille de forme torique (21) dans la bague (9) et par un bourrelet annulaire (22) correspondant à celle-ci au niveau de l'extrémité libre de l'enveloppe (14) de la partie inférieure de l'implant (2).

4. Implant intervertébral (1) selon l'une quelconque des revendications 1 à 3, **caractérisé en ce que** l'enveloppe (14) de la partie inférieure de l'implant (2) présente un évidement radial (15) qui est approprié pour positionner et supporter un pignon en relation fonctionnelle avec la couronne dentée (23).

5. Implant intervertébral (1) selon la revendication 4, **caractérisé en ce que** l'évidement (15) est ininterrompu.

6. Implant intervertébral (1) selon l'une quelconque des revendications 1 à 5, **caractérisé en ce que** pour empêcher une rotation (6) entre la partie supérieure de l'implant (5) et la partie inférieure de l'implant (2), la tige en forme de cylindre circulaire (6) de la partie supérieure de l'implant (5) présente une fente de guidage (16) s'étendant parallèlement à l'axe de rotation (11) et l'enveloppe (14) de la partie inférieure de l'implant (2) présente, sur sa surface interne, un élément de guidage (17) dépassant à l'intérieur, rentrant dans la fente de guidage (16), qui peut de préférence être inséré dans l'enveloppe (14) par une ouverture radiale (18).

7. Implant intervertébral (1) selon l'une quelconque des revendications 1 à 6, **caractérisé en ce que** la tige en forme de cylindre circulaire (6) de la partie supérieure de l'implant (5) présente une plus petite circonférence que la cavité en forme de cylindre creux (3) de la partie inférieure de l'implant (2), de sorte que les deux éléments ne se touchent pas.

8. Implant intervertébral (1) selon l'une quelconque des revendications 1 à 7, **caractérisé en ce que** le pas du filetage (7) et du taraudage (10) se situe dans la gamme de 0,5 à 1,0 mm, de préférence de 0,6 à 0,8 mm.

9. Implant intervertébral (1) selon l'une quelconque des revendications 1 à 8, **caractérisé en ce que** le filetage (7) et le taraudage (10) sont tous deux conçus sous forme de filet à droite.

10. Implant intervertébral (1) selon l'une quelconque des revendications 1 à 9, **caractérisé en ce que** le filetage (7) et le taraudage (10) sont tous deux conçus sous forme de filet multiple, de préférence de filet double.

11. Implant intervertébral (1) selon l'une quelconque des revendications 1 à 10, **caractérisé en ce que** les deux parties d'apposition (4, 8) sont conçues en tant qu'éléments en forme de plaque transversaux à l'axe de rotation (11) présentant des surfaces d'apposition (19, 20) servant à l'appui sur la surface de recouvrement de la vertèbre adjacente.

12. Implant intervertébral (1) selon la revendication 11, **caractérisé en ce que** les surfaces d'apposition (19, 20) ne sont pas disposées orthogonalement à l'axe de rotation (11) et forment de préférence un angle de 83° à 85° avec l'axe de rotation (11).

13. Implant intervertébral (1) selon la revendication 12, **caractérisé en ce que** les deux surfaces d'apposition (19, 20) forment entre elles un angle de 10° à 14°.

14. Implant intervertébral (1) selon l'une quelconque des revendications 11 à 13, **caractérisé en ce qu'**au moins une des deux surfaces d'apposition (19,20), de préférence celle de la partie supérieure de l'implant (5), est convexe.

15. Implant intervertébral (1) selon l'une quelconque des revendications 11 à 13, **caractérisé en ce que** la surface d'apposition (19) de la partie inférieure de l'implant est essentiellement plane.

16. Implant intervertébral (1) selon l'une quelconque des revendications 4 à 15, **caractérisé en ce que** la couronne dentée (23) a une denture droite.
